(19) Europäisches Patentamt
European Patent Office
Office européen des brevets

(11) EP 1 218 341 B1

(12) # EUROPÄISCHE PATENTSCHRIFT

(45) Veröffentlichungstag und Bekanntmachung des Hinweises auf die Patenterteilung:
**24.08.2005 Patentblatt 2005/34**

(21) Anmeldenummer: **00953172.4**

(22) Anmeldetag: **17.08.2000**

(51) Int Cl.[7]: **C07C 311/15**, C07C 317/14, C07D 295/02, C07D 211/14, A61K 31/10, A61K 31/435

(86) Internationale Anmeldenummer:
**PCT/EP2000/008027**

(87) Internationale Veröffentlichungsnummer:
**WO 2001/016094 (08.03.2001 Gazette 2001/10)**

(54) **SULFONYLCARBOXAMIDDERIVATE, VERFAHREN ZU IHRER HERSTELLUNG UND IHRE VERWENDUNG ALS ARZNEIMITTEL**

SULFONYL CARBOXAMIDE DERIVATIVES, METHOD FOR THEIR PRODUCTION AND THEIR USE AS MEDICAMENTS

DERIVES DE SULFONYLCARBOXAMIDE, LEUR PROCEDE DE PRODUCTION ET LEUR UTILISATION COMME MEDICAMENTS

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **01.09.1999 DE 19941540**
**06.06.2000 DE 10027611**

(43) Veröffentlichungstag der Anmeldung:
**03.07.2002 Patentblatt 2002/27**

(73) Patentinhaber: **Aventis Pharma Deutschland GmbH**
**65929 Frankfurt am Main (DE)**

(72) Erfinder:
- **KIRSCH, Reinhard**
  **D-38100 Braunschweig (DE)**
- **SCHAEFER, Hans-Ludwig**
  **D-65239 Hochheim (DE)**
- **FALK, Eugen**
  **D-60529 Frankfurt (DE)**
- **HEMMERLE, Horst**
  **Indianapolis IN 46240 (US)**

(56) Entgegenhaltungen:
**DE-A- 2 145 686** **DE-A- 2 517 183**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 218 341 B1

Printed by Jouve, 75001 PARIS (FR)

## Beschreibung

Die Erfindung betrifft Sulfonylcarboxamiderivate sowie deren physiologisch verträgliche Salze und physiologisch funktionelle Derivate und deren Verwendung zur Herstellung von Medikamenten zur Prävention und Behandlung von Hyperlipidämie sowie arteriosklerotischer Erkrankungen.

In Chemical Abstracts 96, 142393m (1982) wurden bereits Sulfonylcarboxamide beschrieben.
In DE 2145686 wurden bereits 2-Chlor-5-Sulfamylbenzoesäurederivate als Lipidsenker beschrieben.
In DE 2517183 wurden substituierte 5-Sulfamoylanthranilsäurederivate als Blutdrucksenker beschrieben.

Der Erfindung lag die Aufgabe zugrunde, weiter Verbindungen zur Verfügung zu stellen, die eine therapeutisch verwertbare hypolipidämische Wirkung entfalten. In diesem Zusammenhang bestand die Aufgabe insbesondere auch darin, Verbindungen zu finden, bei denen die hypolipidämische Wirkung gegenüber den 2-Chlor-5-Sulfamylbenzoesäurederivaten aus DE 2145686 erhöht ist.

Die Erfindung betrifft daher Verbindungen der Formel I,

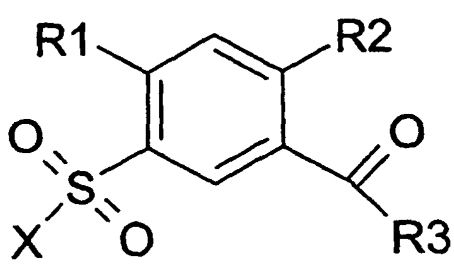

I .

worin bedeuten

R1, R2 unabhängig voneinander NR6R7, Piperidin, Piperazin, Tetrahydropyridin, wobei die Ringe jeweils substituiert sein können mit Phenyl, $(C_1\text{-}C_6)$-Alkyl-Phenyl, $(C_1\text{-}C_6)$-Alkyl, (CO)-$(C_1\text{-}C_6)$-Alkyl;

R6, R7 unabhängig voneinander H, $(C_1\text{-}C_6)$-Alkyl. $(C_1\text{-}C_6)$-Alkyl-NH-$(C_1\text{-}C_6)$-Alkyl, $(C_1\text{-}C_6)$-Alkyl-N-[$(C_1\text{-}C_6)$-Alkyl]$_2$, $(CH_2)_n$-Ar, wobei n = 0 - 6 sein kann und Ar gleich Phenyl, 2-, 3- oder 4-Pyridyl, Piperidinyl, Pyrrolidinyl, 2-, 4- oder 5-Pyrimidinyl, 2-, 3- oder 4-Morpholinyl sein kann und Ar bis zu zweifach substituiert sein kann mit F, Cl, Br, OH, $CF_3$, $NO_2$, CN, $OCF_3$, O-$(C_1\text{-}C_6)$-Alkyl, $(C_1\text{-}C_6)$-Alkyl, COOH, $NH_2$, $(CH_2)_n$-Phenyl, wobei n = 0-3 sein kann;

X $(C_1\text{-}C_6)$-Alkyl, $(CH_2)_n$-Phenyl, wobei n = 0 - 6 sein kann; und der Phenylrest bis zu zweifach substituiert sein kann mit F, Cl, Br, $CF_3$, CN, $OCF_3$, O-$(C_1\text{-}C_6)$-Alkyl, S-$(C_1\text{-}C_6)$-Alkyl, $(C_1\text{-}C_6)$-Alkyl, $(C_3\text{-}C_6)$-Cycloalkyl, COO$(C_1\text{-}C_6)$-Alkyl, COO$(C_3\text{-}C_6)$Cycloalkyl, $CONH_2$, CONH$(C_1\text{-}C_6)$Alkyl, CON[$(C_1\text{-}C_6)$Alkyl]$_2$;

R3 NR10R11, Piperazin;

R10, R11 unabhängig voneinander H, $(C_1\text{-}C_6)$-Alkyl, $(C_3\text{-}C_6)$-Cycloalkyl, CO-$(C_1\text{-}C_6)$-Alkyl, $(C_1\text{-}C_6)$-Alkyl-CO-$(C_1\text{-}C_6)$-Alkyl, $SO_2$-Benzyl, $SO_2$-Benzyl-$OCH_3$, $(CH_2)_n$-Ar, wobei n = 0 - 6 sein kann und Ar gleich Phenyl oder Thienyl sein kann;

sowie deren physiologisch verträgliche Salze.

Die Erfindung bezieht sich auf Verbindungen der Formel I, in Form ihrer Racemate, racemischen Mischungen und reinen Enantiomere sowie auf ihre Diastereomere und Mischungen davon.

Die Alkyl-, Alkenyl- und Alkinylreste in den Substituenten X, R1, R2, R3, R6, R7, R8, R10 und R11 können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der Verbindungen der Formel I sind Salze anorganischer Säuren, wie Salzsäure, Bromwasserstoff-, Phosphor-, Metaphosphor-,

Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isäthion-, Milch-, Lactobion-, Malein-, Apfel-, Methansulfon-, Bernstein-, p-Toluolsulfon-, Wein- und Trifluoressigsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Bevorzugt sind die Salze Methansulfonsäure, Toluolsulfonsäure, Maleinsäure und Phosphorsäure.

Besonders bevorzugt sind die Methansulfonate der Verbindungen der Formel I.

Die Erfindung bezieht sich weiterhin auf physiologisch funktionelle Derivate der Verbindungen der Formel I. Der hier verwendete Begriff "physiologisch funktionelles Derivat" bezeichnet jedes physiologisch verträgliche Derivat einer erfindungsgemäßen Verbindung, z.B. ein Ester, das bei Verabreichung an einen Säuger, wie z.B. den Menschen, in der Lage ist, (direkt oder indirekt) eine solche Verbindung oder einen aktiven Metaboliten hiervon zu bilden.

Ein weiterer Aspekt dieser Erfindung ist die Verwendung von Prodrugs der Verbindungen der Formel I. Solche Prodrugs können in vivo zu einer Verbindung der Formel I metabolisiert werden. Diese Prodrugs können selbst wirksam sein oder nicht.

Die Verbindungen der Formel I können auch in verschiedenen polymorphen Formen vorliegen, z.B. als amorphe und kristalline polymorphe Formen. Alle polymorphen Formen der Verbindungen der Formel I gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeidosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des Salzes der Verbindung der Formel (I). Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%. Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete WirkstoffKonzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die folgenden Zubereitungen dienen zur Erläuterung der Erfindung ohne diese jedoch einzuschränken.

Beispiel A

| Gelatineweichkapseln, enthaltend 100 mg Wirkstoff pro Kapsel: | |
|---|---|
| | pro Kapsel |
| Wirkstoff aus Kokosfett fraktioniertes | 100 mg |
| Triclycerid-Gemisch | 400 mg |
| Kapselinhalt | 500 mg |

Beispiel B

| Emulsion, enthaltend 60 mg Wirkstoff pro 5 ml: | |
|---|---|
| | pro 100 ml Emulsion |
| Wirkstoff | 1,2 g |
| Neutralöl | q.s. |
| Natriumcarboxymethylcellulose | 0,6 g |

(fortgesetzt)

| Emulsion, enthaltend 60 mg Wirkstoff pro 5 ml: | |
|---|---|
| | pro 100 ml Emulsion |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

Beispiel C

| Rektale Arzneiform, enthaltend 40 mg Wirkstoff pro Suppositorium: | |
|---|---|
| | pro Suppositorium |
| Wirkstoff | 40 mg |
| Suppositoriengrundmasse | ad 2 g |

Beispiel D

| Tabletten, enthaltend 40 mg Wirkstoff pro Tablette: | |
|---|---|
| | pro Tablette |
| Lactose | 600 mg |
| Maisstärke | 300 mg |
| lösliche Stärke | 20 mg |
| Magnesiumstearat | 40 mg |
| | 1000 mg |

Beispiel E

| Dragees, enthaltend 50 mg Wirkstoff pro Dragees: | |
|---|---|
| | pro Dragee |
| Wirkstoff | 50 mg |
| Maisstärke | 100 mg |
| Lactose | 60 mg |
| sec. Calciumphosphat | 30 mg |
| lösliche Stärke | 5 mg |
| Magnesiumstearat | 10 mg |
| kolloidale Kieselsäure | 5 mg |
| | 260 mg |

Beispiel F

Für die Herstellung des Inhalts von Hartgelatinekapseln eignen sich die folgenden Rezepturen:

| | | |
|---|---|---|
| a) | Wirkstoff<br>Maisstärke | 100 mg<br>300 mg |

(fortgesetzt)

| | | |
|---|---|---|
| | | 400 mg |
| b) | Wirkstoff | 140 mg |
| | Milchzucker | 180 mg |
| | Maisstärke | 180 mg |
| | | 500 mg |

Beispiel G

Tropfen können nach folgender Rezeptur hergestellt werden (100 mg Wirkstoff in 1 ml = 20 Tropfen):

| | |
|---|---|
| Wirkstoff | 10 g |
| Benzoesäuremethylester | 0,07 g |
| Benzoesäureethylester | 0,03 g |
| Ethanol 96 %ig | 5 ml |
| entimineralisiertes Wasser | ad 100 ml |

Gegenstand der Erfindung ist weiterhin ein Verfahren zur Herstellung der Verbindungen der allgemeinen Formel I, dadurch gekennzeichnet, daß man Verbindungen der allgemeinen Formel I gemäß folgendem Reaktionsschema darstellt:

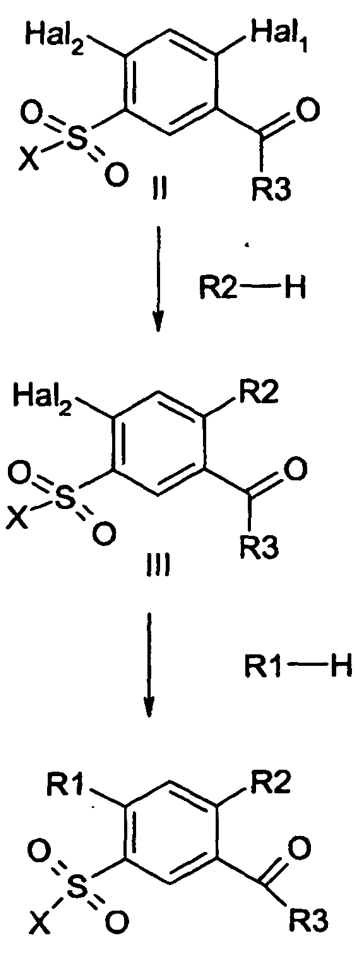

Die nachfolgend aufgeführten Beispiele dienen zur Erläuterung der Erfindung, ohne diese jedoch einzuschränken. Die angegebenen Zersetzungspunkte sind nicht korrigiert und generell von der Aufheizgeschwindigkeit abhängig.

Tabelle 1: Beispiele

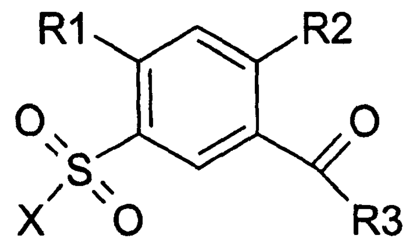

Formel I

| Bsp. | R1 | R2 | R3 | X | Summenformel | Mol-masse | MS $(M+H^+)$ |
|---|---|---|---|---|---|---|---|
| 13 | NH-ethyl-phenyl | Piperazin-1-yl-4-$CH_3$ | $N(CH_3)$-phenyl | $CH_3$ | C28 H34 N4 O3 S | 506,7 | 507,2 |
| 14 | NH-piperidin-4-yl-1-benzyl | Piperazin-1-yl-4-$CH_3$ | $N(CH_3)$-phenyl | $CH_3$ | C32 H41 N5 O3 S | 575,8 | 576,3 |
| 15 | NH-ethyl-phenyl | Piperazin-1-yl-4-$CH_3$ | $N(ethyl)_2$ | $CH_2$-phenyl | C31 H40 N4 O3 S | 548,7 | 549,3 |
| 16 | NH-piperidin-4-yl-1-benzyl | Piperazin-1-yl-4-$CH_3$ | $N(ethyl)_2$ | $CH_2$-phenyl | C35 H47 N5 O3 S | 617,9 | 618,4 |
| 27 | NH-piperidin-4-yl-1-benzyl | Piperazin-1-yl-4-$CH_3$ | Piperidin-1-yl | $CH_3$ | C30 H43 N5 O3 S | 553,8 | 554,3 |
| 28 | Piperazin-1-yl-4-phenyl | Piperazin-1-yl-4-$CH_3$ | Piperidin-1-yl | $CH_3$ | C28 H39 N5 O3 S | 525,7 | 526,3 |
| 29 | NH-butyl-phenyl | Piperazin-1-yl-4-$CH_3$ | Piperidin-1-yl | $CH_3$ | C28 H40 N4 O3 S | 512,7 | 513,3 |
| 30 | Piperidin-1-yl-4-benzyl | Piperazin-1-yl-4-$CH_3$ | Piperidin-1-yl | $CH_3$ | C30 H42 N4 O3 S | 538,8 | 539,3 |
| 31 | NH-$CH_2$-pyrid-2-yl | Piperazin-1-yl-4-$CH_3$ | $N(ethyl)_2$ | $CH_2$-phenyl | C29 H37 N5 O3 S | 535,7 | 536,3 |
| 32 | Piperazin-1-yl-4-phenyl | N(ethyl)-ethyl-$N(CH_3)_2$ | $N(ethyl)_2$ | $CH_2$-phenyl | C34 H47 N5 O3 S | 605,8 | 606,3 |
| 33 | Piperazin-1-yl-4-phenyl | Piperazin-1-yl-4-$CH_3$ | NH-propyl-$OCH_3$ | $CH_3$ | C27 H39 N5 O4 S | 529,7 | 530,3 |

| | | | | | | | |
|---|---|---|---|---|---|---|---|
| 34 | Piperidin-1-yl-4-phenyl | Piperazin-1-yl-4-$CH_3$ | NH-propyl-$OCH_3$ | $CH_3$ | C28 H40 N4 O4 S | 528,7 | 529,3 |
| 35 | Piperidin-1-yl-4-benzyl | Piperazin-1-yl-4-$CH_3$ | NH-propyl-$OCH_3$ | $CH_3$ | C29 H42 N4 O4 S | 542,7 | 543,3 |
| 42 | Piperidin-1-yl-4-phenyl | N(ethyl)-ethyl-N$(CH_3)_2$ | N$(ethyl)_2$ | benzyl | C35 H48 N4 O3 S | 604,9 | 605,3 |
| 46 | 1,2,5,6-Tetrahydropyridin-1-yl-4-phenyl | Piperazin-1-yl-4-$CH_3$ | NH-propyl-$OCH_3$ | $CH_3$ | C28 H38 N4 O4 S | 526,7 | 527,3 |
| 47 | 1,2,5,6-Tetrahydropyridin-1-yl-4-phenyl | Piperazin-1-yl-4-$CH_3$ | Piperidin-1-yl | $CH_3$ | C29 H38 N4 O3 S | 522,7 | 523,3 |
| 53 | Piperidin-1-yl-4-benzyl | NH-ethyl-N-pyrrolidinyl | NH-pentyl | $CH_3$ | C31 H46 N4 O3 S | 554,8 | 555,4 |
| 54 | Piperidin-1-yl-4-phenyl | NH-ethyl-N-pyrrolidinyl | NH-pentyl | $CH_3$ | C30 H44 N4 O3 S | 540,8 | 541,4 |
| 55 | 1,2,5,6-Tetrahydropyridin-1-yl-4-phenyl | NH-ethyl-N-pyrrolidinyl | NH-pentyl | $CH_3$ | C30 H42 N4 O3 S | 538,8 | 539,3 |
| 56 | Piperidin-1-yl-4-benzyl | Piperazin-1-yl-4-$CH_3$ | Piperidin-1-yl | $CH_3$ | C30 H42 N4 O3 S | 538,8 | 539,3 |
| 57 | Piperidin-1-yl-4-benzyl | NH-ethyl-N-pyrrolidinyl | NH-propyl-$OCH_3$ | $CH_3$ | C30 H44 N4 O4 S | 556,8 | 557,4 |
| 58 | Piperidin-1-yl-4-phenyl | NH-ethyl-N-pyrrolidinyl | NH-propyl-$OCH_3$ | $CH_3$ | C29 H42 N4 O4 S | 542,7 | 543,4 |
| 59 | 1,2,5,6-Tetrahydropyridin-1-yl-4-phenyl | NH-ethyl-N-pyrrolidinyl | NH-propyl-$OCH_3$ | $CH_3$ | C29 H40 N4 O4 S | 540,7 | 541,4 |
| 60 | NH-piperidin-4-yl-1-benzyl | NH-ethyl-N-pyrrolidinyl | NH-propyl-$OCH_3$ | $CH_3$ | C30 H45 N5 O4 S | 571,8 | 572,4 |
| 61 | Piperidin-1-yl-4-phenyl | -N(acetyl)-ethyl-N-pyrrolidinyl | NH-propyl-$OCH_3$ | $CH_3$ | C31 H44 N4 O5 S | 584,8 | 585,3 |
| 62 | Piperidin-1-yl-4-benzyl | N(methyl)-ethyl-N$(CH_3)_2$ | NH-propyl-$OCH_3$ | $CH_3$ | C29 H44 N4 O4 S | 544,8 | 545,3 |
| 63 | Piperidin-1-yl-4-phenyl | N(methyl)-ethyl-N$(CH_3)_2$ | NH-propyl-$OCH_3$ | $CH_3$ | C28 H42 N4 O4 S | 530,7 | 531,4 |
| 64 | 1,2,5,6-Tetrahydropyridin-1-yl-4-phenyl | N(methyl)-ethyl-N$(CH_3)_2$ | NH-propyl-$OCH_3$ | $CH_3$ | C28 H40 N4 O4 S | 528,7 | 529,3 |
| 65 | NH-piperidin-4-yl-1-benzyl | N(methyl)-ethyl-N$(CH_3)_2$ | NH-propyl-$OCH_3$ | $CH_3$ | C29 H45 N5 O4 S | 559,8 | 560,4 |
| 66 | Piperidin-1-yl-4-phenyl | N(methyl)-ethyl-N$(CH_3)_2$ | Piperidin-1-yl | $CH_3$ | C29 H42 N4 O3 S | 526,7 | 527,4 |

| | | | | | | | |
|---|---|---|---|---|---|---|---|
| 67 | Piperidin-1-yl-4-benzyl | N(methyl)-ethyl-N($CH_3$)$_2$ | Piperidin-1-yl | $CH_3$ | C30 H44 N4 O3 S | 540,8 | 541,4 |
| 68 | Piperidin-1-yl-4-benzyl | NH-ethyl-N-pyrrolidinyl | Piperidin-1-yl | $CH_3$ | C31 H44 N4 O3 S | 552,8 | 553,4 |
| 69 | NH-piperidin-4-yl-1-benzyl | NH-ethyl-N-pyrrolidinyl | Piperidin-1-yl | $CH_3$ | C31 H45 N5 O3 S | 567,8 | 568,4 |
| 70 | Piperidin-1-yl-4-phenyl | NH-ethyl-N-pyrrolidinyl | N(ethyl)$_2$ | benzyl | C35 H46 N4 O3 S | 602,8 | 603,4 |
| 71 | Piperidin-1-yl-4-phenyl | N(ethyl)-ethyl-N($CH_3$)$_2$ | N(ethyl)$_2$ | benzyl -3-$OCH_3$ | C36 H50 N4 O4 S | 634,9 | 635,4 |
| 72 | Piperidin-1-yl-4-phenyl | N(ethyl)-ethyl-N($CH_3$)$_2$ | N(ethyl)$_2$ | benzyl | C36 H50 N4 O3 S | 618,9 | 619,4 |
| 73 | Piperidin-1-yl-4-(p-F-phenyl) | NH-ethyl-N-pyrrolidinyl | NH-pentyl | benzyl | C36 H47 F N4 O3 S | 634,9 | 635,2 |
| 74 | Piperidin-1-yl-4-(p-$OCH_3$-phenyl) | NH-ethyl-N-pyrrolidinyl | NH-pentyl | benzyl | C37 H50 N4 O4 S | 646,9 | 647,2 |
| 75 | Piperidin-1-yl-4-(p-$CH_3$-phenyl) | NH-ethyl-N-pyrrolidinyl | NH-pentyl | benzyl | C37 H50 N4 O3 S | 630,9 | 631,2 |
| 76 | Piperidin-1-yl-4-(p-F-phenyl) | N(ethyl)-ethyl-N($CH_3$)$_2$ | NH-pentyl | benzyl | C36 H49 F N4 O3 S | 636,9 | 637,2 |
| 77 | Piperidin-1-yl-4-(p-$OCH_3$-phenyl | N(ethyl)-ethyl-N($CH_3$)$_2$ | NH-pentyl | benzyl | C37 H52 N4 O4 S | 648,9 | 649,3 |
| 78 | Piperidin-1-yl-4-(p-$CH_3$-phenyl) | N(ethyl)-ethyl-N($CH_3$)$_2$ | NH-pentyl | benzyl | C37 H52 N4 O3 S | 632,9 | 633,3 |
| 79 | Piperidin-1-yl-4-hydroxy | NH-ethyl-N-pyrrolidinyl | NH-pentyl | benzyl | C30 H44 N4 O4 S | 556,8 | 557,4 |
| 80 | Piperidin-1-yl-4- phenoxy | N(ethyl)-ethyl-N($CH_3$)$_2$ | NH-pentyl | benzyl | C35 H48 N4 O4 S | 620.8 | 621,4 |
| 81 | Piperidin-1-yl-4- phenoxy | NH-ethyl-N-pyrrolidinyl | NH-pentyl | benzyl | C36 H48 N4 O4 S | 632,9 | 633,4 |
| 82 | Piperidin-1-yl-4- phenyl | N(ethyl)-ethyl-N($CH_3$)$_2$ | N(ethyl)$_2$ | 2,4-$F_2$-benzyl | C35 H46 F2 N4 O3 S | 640,8 | 641,4 |
| 83 | Piperidin-1-yl-4- phenyl | NH-ethyl-N-pyrrolidinyl | N(ethyl)$_2$ | 4-$CH_3$-benzyl | C36 H48 N4 O3 S | 616,9 | 617,4 |
| 84 | Piperidin-1-yl-4- phenyl | NH-ethyl-N-pyrrolidinyl | N(ethyl)$_2$ | 3-$OCH_3$-benzyl | C36 H48 N4 O4 S | 632,9 | 633,4 |
| 85 | Piperidin-1-yl-4- phenyl | N(ethyl)-ethyl-N-pyrrolidinyl | N(ethyl)$_2$ | 3-$OCH_3$-benzyl | C38 H52 N4 O4 S | 660,9 | 661,5 |

| | | | | | | | |
|---|---|---|---|---|---|---|---|
| 86 | Piperidin-1-yl-4- benzyl | N(ethyl)-ethyl-N-pyrrolidinyl | $N(ethyl)_2$ | $3-OCH_3$-benzyl | C39 H54 N4 O4 S | 675,0 | 675,5 |
| 87 | Piperidin-1-yl-4- phenyl | NH-propyl-N-(2-oxo-pyrrolidinyl | $N(ethyl)_2$ | benzyl | C36 H46 N4 O4 S | 630,9 | 631,4 |
| 88 | Piperidin-1-yl-4- phenyl | NH-(1-methyl-butyl)-$N(ethyl)_2$ | $N(ethyl)_2$ | benzyl | C38 H54 N4 O3 S | 646,9 | 647,5 |
| 89 | Piperidin-1-yl-4- phenyl | NH-propyl-pyrrolidinyl | $N(ethyl)_2$ | benzyl | C36 H48 N4 O3 S | 616.8 | 617,4 |
| 90 | Piperidin-1-yl-4- phenyl | NH-ethyl-N-pyrrolidinyl | NH(ethyl) | benzyl | C33 H42 N4 O3 S | 574,8 | 575,3 |
| 91 | Piperidin-1-yl-4- phenyl | NH-ethyl-N-pyrrolidinyl | $N(ethyl)_2$ | $3-(OCF_3)$-benzyl | C36 H45 F3 N4 O4 S | 686,8 | 687,2 |
| 92 | Piperidin-1-yl-4- phenyl | NH-ethyl-N-pyrrolidinyl | $N(ethyl)_2$ | 2,4-di-F-benzyl | C35 H44 F2 N4 O3 S | 638,8 | 639,4 |
| 93 | Piperidin-1-yl-4- phenyl | NH-ethyl-N-pyrrolidinyl | $NH_2$ | benzyl | C31 H38 N4 O3 S | 546,7 | 547,3 |
| 94 | Piperidin-1-yl-4- phenyl | NH-ethyl-N-pyrrolidinyl | NH-butyl | benzyl | C35 H46 N4 O3 S | 602,8 | 603,3 |
| 95 | Piperidin-1-yl-4- phenyl | NH-ethyl-N-pyrrolidinyl | NH-hexyl | benzyl | C37 H50 N4 O3 S | 630,9 | 631,3 |
| 96 | Piperidin-1-yl-4-phenyl | N(ethyl)-ethyl-$N(CH_3)_2$ | $N(ethyl)_2$ | $3,5-(OMe_2)$-benzyl | C37 H54 N4 O5 S | 666,2 | 667, |
| 97 | Piperidin-1-yl-4-phenyl | N(ethyl)-ethyl-$N(CH_3)_2$ | $N(ethyl)_2$ | butyl | C32 H50 N4 O3 S | 570,8 | 571,3 |
| 98 | Piperidin-1-yl-4- phenyl | N(ethyl)-ethyl-N-pyrrolidinyl | NH-butyl | benzyl | C37 H50 N4 O3 S | 630,9 | 631,3 |
| 99 | Piperidin-1-yl-4- phenyl | N(ethyl)-ethyl-N-pyrrolidinyl | NH-hexyl | benzyl | C39 H54 N4 O3 S | 658,9 | 659,4 |
| 100 | Piperidin-1-yl-4- phenyl | N(ethyl)-ethyl-N-pyrrolidinyl | NH-ethyl | benzyl | C35 H46 N4 O3 S | 602,8 | 603,2 |
| 101 | Piperidin-1-yl-4- phenyl | N(ethyl)-ethyl-N-pyrrolidinyl | Piperidin-1-yl-4-phenyl | benzyl | C44 H56 N4 O3 S | 718 | 719,3 |
| 102 | NH-propyl-phenyl | NH-ethyl-N-pyrrolidinyl | NH-pentyl | methyl | C28 H42 N4 O3 S | 514,7 | 515,3 |
| 103 | NH-butyl-phenyl | NH-ethyl-N-pyrrolidinyl | NH-pentyl | methyl | C29 H44 N4 O3 S | 528,8 | 529,3 |
| 104 | NH-1-ethyl-$2,2(phenyl)_2$ | NH-ethyl-N-pyrrolidinyl | NH-pentyl | methyl | C33 H44 N4 O3 S | 576,8 | 577,3 |

| | | | | | | | |
|---|---|---|---|---|---|---|---|
| 105 | NH-(p-t-butyl-benzyl) | NH-ethyl-N-pyrrolidinyl | NH-pentyl | methyl | C30 H46 N4 O3 S | 542.8 | 543,4 |
| 106 | Piperidin-1-yl-4- phenyl | NH-ethyl-N-pyrrolidinyl | NH-ethyl | methyl | C27 H38 N4 O3 S | 498.7 | 499,3 |
| 107 | Piperidin-1-yl-4-phenyl | N(ethyl)-ethyl-$N(CH_3)_2$ | NH-pentyl-5-hydroxy | benzyl | C36 H50 N4 O4 S | 634,9 | 635,3 |
| 108 | Piperidin-1-yl-4-phenyl | $N(CH_3)$-ethyl-$NHCH_3$ | NH-pentyl | benzyl | C34 H46 N4 O3 S | 590,8 | 591,3 |
| 109 | Piperidin-1-yl-4-phenyl | $N(CH_3)$-ethyl-$N(CH_3)_2$ | NH-pentyl | benzyl | C35 H48 N4 O3 S | 604,9 | 605,3 |
| 110 | Piperidin-1-yl-4-phenyl | $N(CH_3)$-ethyl-$NHCH_3$ | NH-pentyl-5-OH | benzyl | C34 H46 N4 O4 S | 606,8 | 607,3 |
| 111 | Piperidin-1-yl-4-phenyl | N(ethyl)-ethyl-$N(CH_3)_2$ | $N(ethyl)_2$ | benzyl-3-$OCF_3$ | C36 H47 F3 N4 O4 S | 688,9 | 689,2 |
| 113 | Piperidin-1-yl-4-phenyl | N(ethyl)-ethyl-$N(CH_3)_2$ | $N(ethyl)_2$ | benzyl-4-$CF_3$ | C36 H47 F3 N4 O3 S | 672.9 | 673,3 |
| 114 | NH-butyl-phenyl | NH-ethyl-N-pyrrolidinyl | NH-ethyl | methyl | C26 H38 N4 O3 S | 486,7 | 487,3 |
| 115 | Piperidin-1-yl-4- benzyl | NH-ethyl-N-pyrrolidinyl | NH-ethyl | methyl | C28 H40 N4 O3 S | 512.7 | 513,3 |
| 116 | Piperidin-1-yl-4-phenyl | N(ethyl)-ethyl-$N(CH_3)_2$ | $N(ethyl)_2$ | benzyl-4-cyano | C36 H47 N5 O3 S | 629,9 | 630,3 |
| 117 | NH-1-ethyl-2,2$(phenyl)_2$ | N(ethyl)-ethyl-$N(CH_3)_2$ | $N(ethyl)_2$ | benzyl-4-$CF_3$ | C39 H47 F3 N4 O3 S | 708,9 | 709,0 |
| 118 | NH-butyl-phenyl | N(ethyl)-ethyl-$N(CH_3)_2$ | $N(ethyl)_2$ | benzyl-4-cyano | C35 H47 N5 O3 S | 617,9 | 618,3 |
| 119 | NH-butyl-phenyl | N(methyl)-ethyl-N-pyrrolidinyl | NH-pentyl | methyl | C30 H46 N4 O3 S | 542,8 | 543,3 |
| 120 | Piperidin-1-yl-4-phenyl | N(methyl)-ethyl-NH-ethyl | NH-pentyl | benzyl | C35 H48 N4 O3 S | 604,9 | 605,3 |
| 121 | 1,2,3,4-tetrahydro-isochinolin-2-yl | N(methyl)-ethyl-N-pyrrolidinyl | NH-pentyl | methyl | C29 H42 N4 O3 S | 526,7 | 527,3 |
| 122 | 1,2,3,4-tetrahydro-isochinolin-2-yl | NH-ethyl-N-pyrrolidinyl | $N(ethyl)_2$ | benzyl-3-$OCH_3$ | C34 H44 N4 O4 S | 604,8 | 605,3 |
| 123 | Piperidin-1-yl-4-benzyl | N(ethyl)-ethyl-$N(CH_3)_2$ | $N(ethyl)_2$ | benzyl-4-$CH_3$ | C37 H52 N4 O3 S | 632,9 | 633,4 |
| 124 | 1,2,3,4-tetrahydro-isochinolin-2-yl | N(ethyl)-ethyl-$N(CH_3)_2$ | $N(ethyl)_2$ | benzyl-4-$CH_3$ | C34 H46 N4 O3 S | 590,8 | 591,5 |

| | | | | | | | |
|---|---|---|---|---|---|---|---|
| 125 | 1,2,3,4-tetrahydro-isochinolin-2-yl | NH-ethyl-N-pyrrolidinyl | NH-propyl-$OCH_3$ | methyl | C27 H38 N4 O4 S | 514,7 | 515,3 |
| 126 | 1,2,3,4-tetrahydro-isochinolin-2-yl | N(ethyl)-ethyl-N-pyrrolidinyl | $N(ethyl)_2$ | benzyl-3-$OCH_3$ | C36 H48 N4 O4 S | 632,9 | 633,6 |
| 127 | NH-1-ethyl-2,2$(phenyl)_2$ | NH-ethyl-N-pyrrolidinyl | NH-propyl-$OCH_3$ | methyl | C32 H42 N4 O4 S | 578,8 | 579,3 |
| 128 | Piperidin-1-yl-4- phenyl | NH-ethyl-N-pyrrolidinyl | NH-butyl | benzyl-4-$CH_3$ | C36 H48 N4 O3 S | 616,9 | 617,6 |
| 129 | Piperidin-1-yl-4- phenyl | N(butyl)-ethyl-N-pyrrolidinyl | NH-pentyl | methyl | C34 H52 N4 O3 S | 596,9 | 597,3 |
| 130 | Piperidin-1-yl-4- phenyl | N(butyl)-ethyl-N-pyrrolidinyl | $N(ethyl)_2$ | methyl | C33 H50 N4 O3 S | 582,9 | 583,2 |
| 131 | Piperidin-1-yl-4- phenyl | NH-propyl-N-pyrrolidinyl | $N(butyl)_2$ | methyl | C34 H52 N4 O3 S | 596,9 | 597,2 |
| 132 | Piperidin-1-yl-4- phenyl | N(ethyl)-ethyl-N-pyrrolidinyl | $N(butyl)_2$ | methyl | C35 H54 N4 O3 S | 610,9 | 611,2 |
| 133 | Piperidin-1-yl-4- phenyl | NH-ethyl-N-piperidinyl | NH(pentyl) | methyl | C31 H46 N4 O3 S | 554,8 | 555,3 |
| 134 | Piperidin-1-yl-4- phenyl-4-$NH_2$ | NH-ethyl-N-pyrrolidinyl | NH-pentyl | methyl | C30 H45 N5 O3 S | 555,8 | 556,3 |
| 135 | Piperidin-1-yl-4- phenyl | NH-ethyl-N-pyrrolidinyl | NH-butyl | benzyl-3,5$(OCH_3)_2$ | C37 H50 N4 O5 S | 662,9 | 663,6 |
| 136 | Piperidin-1-yl-4-(4-$NH_2$-phenyl) | N(butyl)-ethyl-N-pyrrolidinyl | $N(ethyl)_2$ | methyl | C33 H51 N5 O3 S | 597,9 | 598,5 |
| 137 | Piperidin-1-yl-4-(4-$NH_2$-phenyl) | N(ethyl)-ethyl-N-pyrrolidinyl | NH-butyl | methyl | C31 H47 N5 O3 S | 569,8 | 570,4 |
| 138 | Piperidin-1-yl-4-phenyl | NH-ethyl-N-pyrrolidinyl | NH-hexyl | methyl | C31 H46 N4 O3 S | 554,8 | 555,4 |
| 139 | Piperidin-1-yl-4-phenyl | NH-ethyl-N-pyrrolidinyl | NH-pentyl | benzyl-3-$NH_2$ | C36 H49 N5 O3 S | 631,9 | 632,4 |
| 140 | Piperidin-1-yl-4-phenyl | N(ethyl)-ethyl-$N(CH_3)_2$ | NH-pentyl-$NH_2$ | benzyl | C36 H51 N5 O3 S | 633,9 | 634,4 |

Beispiel 141: Methansulfonat von Beispiel 54

2-[NH-ethyl-N-pyrrolidinyl]-NH-penthyl-5-methylsulfonyl-4-(4-phenyl-piperidin-1-yl)benzamid

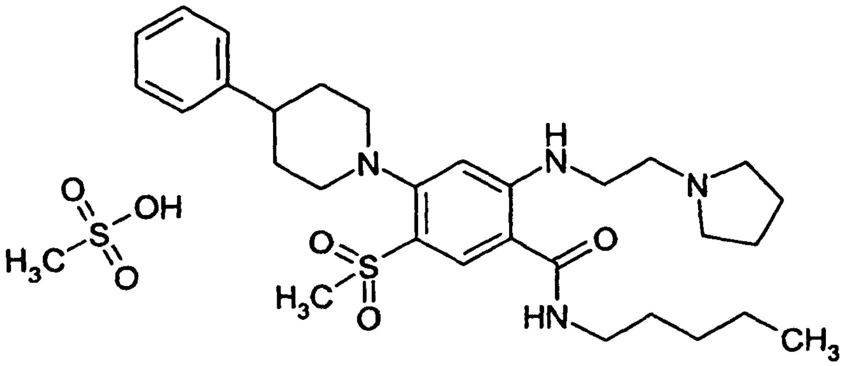

20,0 g der freien Base aus Beispiel 54 werden in heißem Isopropanol gelöst und mit 5,1 ml Methansulfonsäure versetzt. Nach Abkühlung der Lösung auf Zimmertemperatur wird die enstandene Suspension eine weitere Stunde bei einer Temperatur zwischen 0°C und 5°C gerührt und dann das Produkt mittels Filtration gewonnen. Das Rohprodukt wird aus 200 ml Isopropanol umkristallisiert und im Vakuum bei 50°C getrocknet. Man erhält 21,0 g des Salzes vom Schmelzpunkt 205°C bis 208°C.

Beispiel 142: Toluolsulfonat von Beispiel 54

2-[NH-ethyl-N-pyrrolidinyl]-NH-penthyl-5-methylsulfonyl-4-(4-phenyl-piperidin-1-yl)benzamid

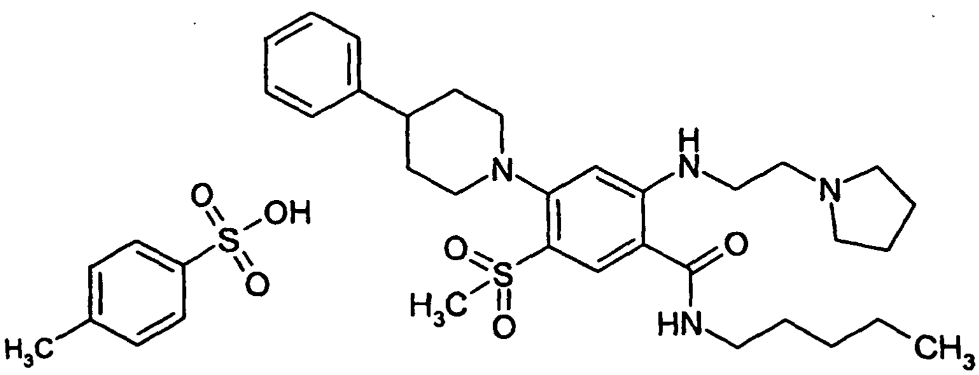

Beispiel 143: Maleinat von Beispiel 54

2-[NH-ethyl-N-pyrrolidinyl]-NH-penthyl-5-methylsulfonyl-4-(4-phenyl-piperidin-1-yl)benzamid

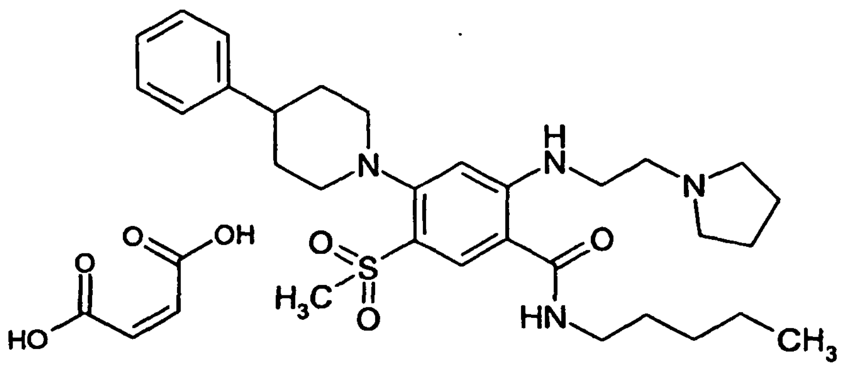

Die Verbindungen der Formel I zeichnen sich durch günstige Wirkungen auf den Fettstoffwechsel aus, insbesondere sind sie als Hypolipidämika geeignet. Die Verbindungen können allein oder in Kombination mit weiteren Lipidsenkern eingesetzt werden. Solche weiteren Lipidsenker werden z.B. in der Roten Liste, Kapitel 58 genannt. Die Verbindungen eignen sich zur Prophylaxe sowie insbesondere zur Behandlung von Hyperlipidämie.
Arteriosklerose ist eine komplexe Erkrankung des Stoffwechsel- und Kreislaufsystems. Erhöhtes Plasma-LDL-Cholesterin ist einer der Hauptrisikoparameter dieser Erkrankung. Beim Menschen wird LDL-Cholesterin zum überwiegenden Teil über den LDL-Rezeptor in der Leber aus dem Blutkreislauf entfernt. Eine Senkung des LDL-Plasmacholesterins senkt das Arteriosklerosirisiko und damit auch die Gesamtmortalität. Die erfindungsgemäßen Verbindungen eignen sich somit auch zur Prophylaxe und zur Behandlung arteriosklerotischer Erkrankungen.
Die Wirksamkeit der Verbindungen wurde wie folgt getestet:

1) In-vitro-Bestimmung des LDL-Rezeptorinduktion mittels des Luziferase-Assays

Die LDL-Rezeptorinduktion wird mittels des Luziferase Tests folgendermaßen bestimmt: Dazu wird ein regulatorisches DNA Fragment (4kb) des humanen LDL-Rezeptors Gens , daß die komplette Promotorregion enthält, an das Luziferase-Reporter-Gen aus der Feuerfliege gekoppelt und stabil in eine Hep-G2 Zelllinie transfiziert. Zellen aus dieser Linie wurden auf Kollagen beschichteten 96 Well Platten in MEM (Minimum Esential Medium) ausgesäht. Nach 24 Stunden in Kultur wurden die Testsubstanzen, gelöst in DMSO, in Endkonzentrationen von 10 nM bis 10 μM zugegeben (DMSO Endkonzentration = 2%). Die Substanzen wurden über Nacht für 12-18 Stunden inkubiert (jeweils 4 Wells/Konz.), danach wurde D-Luziferin als Substrat für die Luziferase zugegeben und die Lumineszens gemessen. Die gemessene Luminiszens in prozentualer Relation zu Kontrolle gesetzt (Kontrolle = 100%), die nur mit DMSO inkubiert wurde, ergibt das Maß für die relative LDL-Rezeptorinduktion (Tabelle 2).
Weitere Details der Methode sind beschrieben in: Current Ptotocols in Molecular Biology, F.M. Ausubel, R. Brent, R. E.Kingston, D.D. Moore, J.G.Seidman, J.A.Smith and Kevin Struhl editors, J.Wiley and Sons Inc., U.S.A.

Tabelle 2:

| LDL-Rezeptor Induktion ausgewählter Beispiel in % gegenüber der Kontrolle | |
|---|---|
| Beispiel | LDL-Rezeptor Induktion (% gegen Kontrolle); jeweilige Konzentration der Testverbindung in μM in () |
| 14 | 176 % (1,5 μM) |
| 30 | 255 % (1,5 μM); 199 % (0,15 μM) |
| 34 | 291 % (4 μM); 210 % (0,15 μM) |
| 42 | 149 % (0,05 μM) |

Tabelle 2: (fortgesetzt)

| LDL-Rezeptor Induktion ausgewählter Beispiel in % gegenüber der Kontrolle | |
|---|---|
| Beispiel | LDL-Rezeptor Induktion (% gegen Kontrolle); jeweilige Konzentration der Testverbindung in µM in () |
| 53 | 221 % (0,15 µM); 197 % (0,05 µM) |
| 54 | 223 % (0,15 µM) |
| 57 | 222 % (0,15 µM) |
| 62 | 205 % (0,05 µM) |
| 67 | 203 % (0,15 µM) |
| 76 | 244 % (0,15 µM) |
| 78 | 200 % (0,15 µM) |
| 80 | 239 % (0,15 µM) |
| 82 | 227 % (0,15 µM) |
| 83 | 212 % (0,15 µM) |
| 84 | 240 % (0,05 µM) |
| 91 | 231 % (0,05 µM) |
| 96 | 196 % (0,05 µM) |
| 105 | 236 % (0,05 µM) |
| 107 | 288 % (0,05 µM) |
| 113 | 217 % (0,05 µM) |
| 116 | 235 % (0,05 µM) |
| 141 | 280 % (0,15 µM) |
| 142 | 281 % (0,15 µM) |
| 143 | 308 % (0,15 µM) |

2) In vivo-Bestimmung der LDL-Cholesterinsenkung am Hamster. Cholesterinsenkenden Wirkung von LDL-Rezeptorinduktoren bei hyperlipämischen Hamstern

In diesem Tierversuch wurde die Wirkung der LDL-Rezeptorinduktoren nach Bolusapplikation am fettgefütterten Hamster untersucht.

Als Versuchstiere wurden männliche syrische Hamster (Charles River) mit einem durchschnittlichen Körpergewicht von 100 - 120 g zu Adaptionsbeginn verwendet. Die Tiere wurden anhand des Körpergewichtes in Gruppen (n = 6) eingeteilt. Durch Fütterung einer mit 15 % Butter und 3 % Cholesterin angereicherten Diät wurde eine starke Hyperlipidämie induziert. Nach 2 wöchiger Vorfütterung begann die Behandlung. Die Testsubstanzen wurden oral mit einer Schlund-Magensode über einen Zeitraum von 10 Tagen 1 mal täglich appliziert. Nach 10 Tagen wurde der Plasma Lipidspiegel analysiert.

Tabelle 3 zeigt die relativen Veränderungen des Lipidspiegels im Vergleich zu Plazebo behandelten Kontrolltieren in %.

Tabelle 3:

| Relative prozentuale Veränderung des Plasma-Lipidspiegels bei hyperlipidämischen Hamstern nach zehntägiger oraler Behandlung [%]. | | | | |
|---|---|---|---|---|
| Gruppe | Behandlung (Bsp.-Nr./ Dosis) | Total-Cholesterin | LDL-Cholesterin | Triglyceride |
| 1 | Kontrolle I | - | - | - |

Tabelle 3: (fortgesetzt)

| Relative prozentuale Veränderung des Plasma-Lipidspiegels bei hyperlipidämischen Hamstern nach zehntägiger oraler Behandlung [%]. | | | | |
|---|---|---|---|---|
| Gruppe | Behandlung (Bsp.-Nr./ Dosis) | Total-Cholesterin | LDL-Cholesterin | Triglyceride |
| 2 | 37<br>20 mg/kg p.o. | - 21 | - 31 | - 46 |
| 3 | 53<br>10 mg/kg p.o. | -7 | -52 | +21 |
| 4 | 53<br>20 mg/kg p.o. | -16 | -57 | -11 |
| 5 | 53<br>40 mg/kg p.o. | - 17 | - 58 | - 23 |
| | | | | |

Aus der deutlichen Senkung von Total-Cholesterin, LDL-Cholesterin und Triglyceriden ist die gute lipidsenkende Wirkung der erfindungsgemäßen Verbindungen abzulesen.

Es wurde ein Vergleichsversuch mittels des oben beschriebenen Luziferase-Assays mit 2,4-Dichlor-5-(3,5-dimethylpiperidinosulfamoyl)-benzoesäure und der Verbindung aus Beispiel 42 durchgeführt.

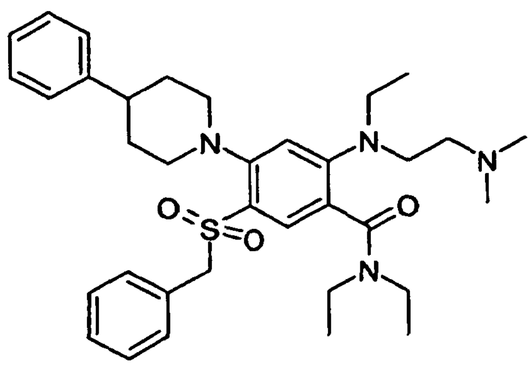

Beispiel 42

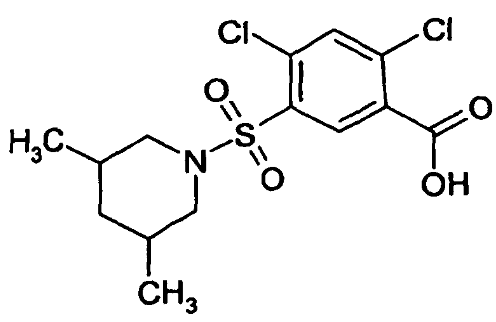

2,4-Dichlor-5-(3,5-dimethylpiperidinosulfamoyl)-benzoesäure

Luziferase Assay:
LDL-Rezeptor Induktion in % gegenüber der Kontrolle

| Substanz | 4µM | 1.5 µM | 0.15 µM | 0.05 µM |
|---|---|---|---|---|
| Beispiel 42 | 278 | 250 | 219 | 204 |
| 2,4-Dichlor-5-(3,5-dimethylpiperidinosulfamoyl)-benzoesäure | 100 | 90 | 90 | 94 |

Die erfindungsgemäßen Verbindungen der Formel I zeigen damit eine deutlich verbesserte Wirkung gegenüber 2,4-Dichlor-5-(3,5-dimethyl-piperidinosulfamoyl)-benzoesäure.

Zur näheren Erläuterung der Herstellung ist nachfolgend ein Beispiel (Nr. 42) genau beschrieben.

Ausführungsbeispiel:

2-[(2-Dimethylamino-ethyl)-ethyl-amino]-N,N-diethyl-5-phenylmethansulfonyl-4-(4-phenyl-piperidin-1-yl)-benzamid (Tabelle 1, Beispiel 42)

1. Herstellung von 3-Chlorsulfonyl-4-chlor-6-fluor-benzoesäure

20 g (0,115 mol) 4-Chlor-2-fluorbenzoesäure werden bei 20°C unter Rühren portionsweise in 100 ml Chlorsulfonsäure eingetragen. Die Reaktionsmischung wird unter Rühren 5 Stunden auf 120 °C erhitzt. Zur Aufarbeitung wird die erkaltete Reaktionsmischung tropfenweise unter kräftigem Rühren in 5 l Eis/Wasser-Gemisch eingetragen. Der gebildete Niederschlag wird abgesaugt, mit Wasser gewaschen und anschließend im Vakuumtrockenschrank 1 Stunde bei 50 °C getrocknet.

Man erhält 61,5 g 3-Chlorsulfonyl-4-chlor-6-fluor-benzoesäure, farblose Kristalle vom Schmelzpunkt 135 °C

2. Herstellung von 5-Carboxy-2-chlor-4-fluor-benzosulfinsäure-bis-Natriumsalz

71g (0,563 mol; 2,5 Äquivalente) Natriumsulfit werden in 200 ml Wasser gelöst und unter Eiskühlung mit 61,5 g der Verbindung aus Vorschrift 1. (3-Chlorsulfonyl-4-chlor-6-fluor-benzoesäure) versetzt. Es wird mittels Zugabe von konz. wässriger Natronlauge der pH-wert der Lösung auf pH 9 eingestellt und 6 Stunden bei 20 °C gerührt Anschließend wird mittels konz. wässriger Salzsäure auf pH 1 angesäuert, wobei die anfallende Sulfinsäure ausfällt. Nach Abfiltration der Sulfinsäure wird das Reaktionsprodukt in 600 ml Wasser gelöst und der pH-Wert der Lösung durch Zugabe von konz. wässriger NaOH auf pH 10 eingestellt. Nach Filtration über Aktivkohle und Entfernen des Lösungsmittels im Vakuum wird der ölige Rückstand durch Zusatz von 100 ml Aceton zur Kristallisation gebracht.

Man erhält 59,2g farblose Kristalle (93 % d. Th), welche direkt weiter umgesetzt werden.

3. Herstellung von 4-Chlor-2-fluor-5-phenylmethansulfonyl-benzoesäurebenzylester

28,3 g (0,1 mol) der unter 2. hergestellten Verbindung werden in 250 ml N-Methyl-2-pyrrolidon suspendiert und nacheinander mit 41 g (0,24 mol Benzylbromid) und 4,6 g (0,3 mol) Kaliumcarbonat versetzt. Die Reaktionsmischung wird 8 Stunden bei 60 °C gerührt. Zur Aufarbeitung wird das Reaktionsgemisch nach Abkühlung auf Zimmertemperatur auf 1,5 Liter Eiswasser gegeben, wobei das Reaktionsprodukt nach 20 Minuten Zeit in Form eines farblosen Feststoffes ausfällt, welcher abfiltriert wird.

Man erhält 38,9 g (93 % derTheorie) 4-Chlor-2-fluor-5-phenylmethansulfonylbenzoesäure-benzylester; die Verbindung wird direkt, ohne weitere Reinigungsschritte nach 4. umgesetzt.

4. Herstellung von 4-Chlor-2-fluor-5-phenylmethanesulfonyl-benzoesäure

1,26 g (1,2 Äquivalente) NaOH-Plätzchen werden in 40 ml Wasser gelöst und mit 11 g (26,3 mmol)) 4-Chlor-2-fluor-5-phenylmethansulfonyl-benzoesäurebenzylester, gelöst in 40 ml Tetrahydrofuran, versetzt. Die Reaktionslösung wird unter Rühren 3 Stunden bei 20 °C belassen.

Anschließend wird zur Aufarbeitung auf 1 l Eis/Wasser-Mischung gegossen und mittels Zusatz von konz. wässriger Salzsäure auf pH 1,2 eingestellt. Das Reaktionsprodukt fällt nach einiger Zeit in Form farbloser Kristalle aus. Man erhält 8,4 g (97,7 % d.Th) vom Schmelzpunkt 180 bis 184 °C.

5. Herstellung von 4-Chlor-N,N-diethyl-2-fluor-5-phenylmethansulfonyl-benzamid

6,6 g (20 mmol) der Carbonsäure aus Beispiel 4. werden in 50 ml Thionylchlorid suspendiert und unter Rühren 1 Stunde unter Rückfluß erhitzt. Anschließend wird am Rotationsverdampfer unter reduziertem Druck eingeengt, der

ölige Rückstand in 100 ml absolutem Dichlormethan gelöst und bei -10 °C tropfenweise mit 3,1 g (2,1 Äquivalente) Diethylamin versetzt. Nach beendeter Zugabe wird noch 1 Stunde bei 20°C gerührt. Die Reaktionsmischung wird anschließend mehrmals sukcessive mit gesättigter, wässriger Bicartionatlösung und Wasser gewaschen, mittels Natriumsulfat getrocknet und das Lösungsmittel im Vakuum am Rotationsverdampfer entfernt. Das auf diese Weise erhaltene Rohprodukt wird mittels Chromatographie an Kieselgel (40 -63µ Komgröße, Fa. Merck Darmstadt) mit n-Heptan/Ethylacetat 1:1 als mobiler Phase gereinigt ($R_F$= 0,52).

Man erhält nach Entfernen der mobilen Phase am Rotationsverdampfer unter reduziertem Druck 7,7 g 4-Chlor-N,N-diethyl-2-fluor-5-phenylmethanesulfonylbenzamid (Ausbeute quantitativ).

6. Herstellung von 4-Chloro-2-[(2-dimethylamino-ethyl)-ethyl-amino]-N,N-diethyl-5-phenylmethansulfonyl- benzamid

5,7 g (15 mmol) 4-Chlor-N,N-diethyl-2-fluor-5-phenylmethansulfonyl-benzamid werden in 50 ml Ethanol gelöst und nach Zusatz von 2,6 g (22,5 mmol; 1,5 Äquivalente) N,N-Dimethyl-ethylendiamin 18 Stunden unter Rückfluß erhitzt. Anschließend wird unter das Lösungsmittel reduziertem Druck entfernt, der Rückstand mit 100 ml Dichlormethan aufgenommen und mit gesättigter wässriger Bicarbonantlösung, gefolgt von mehrmaliger Extraktion mit jeweils 30 ml Wasser, gewaschen. Die organische Phase wird anschließend über Natriumsulfat getrocknet und das Lösungsmittel im Vakuum am Rotations-verdampfer entfernt.

Man erhält 7,3 g hellgelbes Öl, welches direkt zum Endprodukt der Reaktionsfolge umgesetzt wird (s. Vorschrift 7).

7. Herstellung von 2-[(2-Dimethylamino-ethyl)-ethyl-amino]-N,N-diethyl-5-phenylmethansulfonyl-4-(4- phenyl-piperidin-1-yl)-benzamid - (Beisp. 42)

3,5 g (7,3 mmol) 4-Chloro-2-[(2-dimethylamino-ethyl)-ethyl-amino]-N,N-diethyl-5-phenylmethansulfonyl- benzamid aus Versuchsbeschreibung 6. werden mit 5.9 g 4-Phenylpiperidin (5 Äqiuvalente), hergestellt mittels Hydrierung von käuflichem 4-Phenyl-1,2,3,6-tetrahydro-pyridin, vermischt und 5 Stunden bei 150 °C gerührt. Anschließend wird in 150 ml Dichlormethan gelöst und mit gesättigter wässriger Natriumbicarbonat-Lösung sowie Wasser ausgeschüttelt. Nach Trocknung der organischen Phase über Natriumsulfat wird das Lösungsmittel unter reduziertem Druck am Rotationsverdampfer entfernt. Zur Reinigung des Rohproduktes wird unter Verwendung von Ethylacetat/Methanol, Mischungsverhältnis 2:1, an Kieselgel (40 -63µ Korngröße, Fa. Merck Darmstadt) als stationärer Phase chromatographiert.

Man erhält 4,5 g 2-[(2-Dimethylamino-ethyl)-ethyl-amino]-N,N-diethyl-5-phenylmethansulfonyl-4-(4- phenylpiperidin-1-yl)-benzamid, hellgelbes Öl

MS: C35 H 48 N4 03 S (604,9); Massenspektrum 605,3 (M + $H^+$)

## Patentansprüche

1. Verbindungen der Formel I,

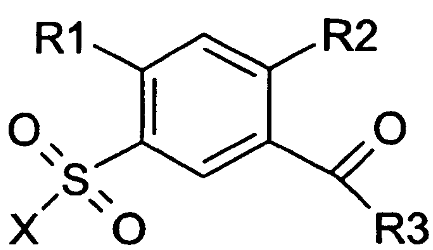

I

worin bedeuten

R1, R2 unabhängig voneinander NR6R7, Piperidin, Piperazin, Tetrahydropyridin, wobei die Ringe jeweils

substituiert sein können mit Phenyl, $(C_1-C_6)$-Alkyl-Phenyl, $(C_1-C_6)$-Alkyl, (CO)-$(C_1-C_6)$-Alkyl;

R6, R7 unabhängig voneinander H, $(C_1-C_6)$-Alkyl. $(C_1-C_6)$-Alkyl-NH-$(C_1-C_6)$-Alkyl, $(C_1-C_6)$-Alkyl-N-[$(C_1-C_6)$-Alkyl]$_2$, $(CH_2)_n$-Ar, wobei n = 0 - 6 sein kann und Ar gleich Phenyl, 2-, 3- oder 4-Pyridyl, Piperidinyl, Pyrrolidinyl, 2-, 4- oder 5-Pyrimidinyl, 2-, 3- oder 4-Morpholinyl sein kann und Ar bis zu zweifach substituiert sein kann mit F, Cl, Br, OH, $CF_3$, $NO_2$, CN, $OCF_3$, O-$(C_1-C_6)$-Alkyl, $(C_1-C_6)$-Alkyl, COOH, $NH_2$, $(CH_2)_n$-Phenyl, wobei n = 0-3 sein kann;

X $(C_1-C_6)$-Alkyl, $(CH_2)_n$-Phenyl, wobei n = 0 - 6 sein kann; und der Phenylrest bis zu zweifach substituiert sein kann mit F, Cl, Br, $CF_3$, CN, $OCF_3$, O-$(C_1-C_6)$-Alkyl, S-$(C_1-C_6)$-Alkyl, $(C_1-C_6)$-Alkyl, $(C_3-C_6)$-Cycloalkyl, COO$(C_1-C_6)$-Alkyl, COO$(C_3-C_6)$Cycloalkyl, $CONH_2$, CONH$(C_1-C_6)$Alkyl, CON[$(C_1-C_6)$Alkyl]$_2$;

R3 NR10R11, Piperazin;

R10, R11 unabhängig voneinander H, $(C_1-C_6)$-Alkyl, $(C_3-C_6)$-Cycloalkyl, CO-$(C_1-C_6)$-Alkyl, $(C_1-C_6)$-Alkyl-CO-$(C_1-C_6)$-Alkyl, $SO_2$-Benzyl, $SO_2$-Benzyl-$OCH_3$, $(CH_2)_n$-Ar, wobei n = 0 - 6 sein kann und Ar gleich Phenyl oder Thienyl sein kann;

sowie deren physiologisch verträgliche Salze.

2. Arzneimittel enthaltend eine oder mehrere der Verbindungen gemäß Anspruch 1.

3. Arzneimittel enthaltend eine oder mehrere der Verbindungen gemäß Anspruch 1 und einen oder mehrere Lipidsenker.

4. Verbindungen gemäß Anspruch 1 zur Anwendung als Medikament zur Behandlung der Hyperlipidämie.

5. Verbindungen gemäß Anspruch 1 zur Anwendung als Medikament zur Behandlung der Arteriosklerose.

6. Verfahren zur Herstellung eines Arzneimittels enthaltend eine oder mehrere der Verbindungen gemäß Anspruch 1, **dadurch gekennzeichnet, daß** der Wirkstoff mit einem pharmazeutisch geeigneten Träger vermischt wird und diese Mischung in eine für die Verabreichung geeignete Form gebracht wird.

7. Verwendung der Verbindungen gemäß Anspruch 1 zur Herstellung eines Medikaments zur Behandlung der Hyperlipidämie.

8. Verwendung der Verbindungen gemäß Anspruch 1 zur Herstellung eines Medikaments zur Behandlung der Arteriosklerose.

## Claims

1. Compounds of the formula I

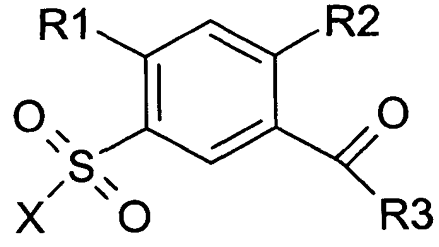

I

in which

R1, R2 are, independently of one another, NR6R7, piperidine, piperazine, tetrahydropyridine, it being possible for each of the rings to be substituted by phenyl, ($C_1$-$C_6$)-alkylphenyl, ($C_1$-$C_6$)-alkyl, (CO)-($C_1$-$C_6$)-alkyl;

R6, R7 are, independently of one another, H, ($C_1$-$C_6$)-alkyl, ($C_1$-$C_6$)-alkyl-NH-($C_1$-$C_6$)-alkyl, ($C_1$-$C_6$)-alkyl-N-[($C_1$-$C_6$)-alkyl]$_2$, $(CH_2)_n$-Ar, where n can be 0 - 6 and Ar can be equal to phenyl, 2-, 3- or 4-pyridyl, piperidinyl, pyrrolidinyl, 2-, 4- or 5-pyrimidinyl, 2-, 3- or 4-morpholinyl and Ar can be substituted up to two times by F, Cl, Br, OH, $CF_3$, $NO_2$, CN, $OCF_3$, O-($C_1$-$C_6$)-alkyl, ($C_1$-$C_6$)-alkyl, COOH, $NH_2$, $(CH_2)_n$-phenyl, where n can be 0-3;

X is ($C_1$-$C_6$)-alkyl, $(CH_2)_n$-phenyl, where n can be 0 - 6; and the phenyl radical can be substituted up to two times by F, Cl, Br, $CF_3$, CN, $OCF_3$, O-($C_1$-$C_6$)-alkyl, S-($C_1$-$C_6$)-alkyl, ($C_1$-$C_6$)-alkyl, ($C_3$-$C_6$)-cycloalkyl, COO($C_1$-$C_6$)-alkyl, COO($C_3$-$C_6$)cycloalkyl, $CONH_2$, CONH($C_1$-$C_6$)alkyl, CON[($C_1$-$C_6$)alkyl]$_2$;

R3 is NR10R11 or piperazine;

R10, R11 are, independently of one another, H, ($C_1$-$C_6$)-alkyl, ($C_3$-$C_6$)-cycloalkyl, CO-($C_1$-$C_6$)-alkyl, ($C_1$-$C_6$)-alkyl-CO-($C_1$-$C_6$)-alkyl, $SO_2$-benzyl, $SO_2$-benzyl-$OCH_3$, $(CH_2)_n$-Ar, where n can be 0 - 6 and Ar can be phenyl or thienyl;

and their physiologically acceptable salts.

2. Pharmaceutical comprising one or more compounds according to Claim 1.

3. Pharmaceutical comprising one or more compounds according to Claim 1 and one or more lipid-lowering agents.

4. Compounds according to in Claim 1 for use as a medicine for the treatment of hyperlipidaemia.

5. Compounds according to in Claim 1 for use as a medicine for the treatment of arteriosclerosis.

6. Process for preparing a pharmaceutical comprising one or more of the compounds according to Claim 1, **characterized in that** the active substance is mixed with a pharmaceutically suitable carrier and said mixture is brought into a form suitable for administration.

7. Use of the compounds according to Claim 1 for preparing a medicine for the treatment of hyperlipidaemia.

8. Use of the compounds according to Claim 1 for preparing a medicine for the treatment of arteriosclerosis.

## Revendications

1. Composés de formule I,

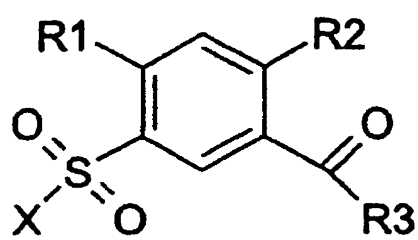

I

dans laquelle

R1, R2 représentent, indépendamment l'un de l'autre NR6R7, une pipéridine, une pipérazine, une tétraydropyridine, où les noyaux peuvent chacun être substitués par un phényle, un alkylphényle en $C_1$-$C_6$, un alkyle en $C_1$-$C_6$, un (CO)-($C_1$-$C_6$)alkyle ;

R6, R7 représentent indépendamment l'un de l'autre, H, un alkyle en $C_1$-$C_6$, un ($C_1$-$C_6$)alkyl-NH-($C_1$-$C_6$)alkyle, un ($C_1$-$C_6$)-alkyl-N-[($C_1$-$C_6$)alkyle]$_2$, un $(CH_2)_n$-Ar, où n peut valoir de 0 à 6 et Ar peut être un phényle, un 2-, un 3- ou un 4-pyridyle, un pipéridynyle, un pyrrolidinyle, un 2-, un 4- ou un 5-pyrimidinyle, un 2-, un 3- ou un 4-morpholinyle et Ar peut être jusqu'à bi-substitué par F, Cl, Br, OH, $CF_3$, $NO_2$, CN, $OCF_3$, un O-($C_1$-$C_6$)alkyle, un alkyle en $C_1$-$C_6$, COOH, $NH_2$, un $(CH_2)_n$-phényle, où n peut valoir de 0 à 3 ;

X représente un alkyle en $C_1$-$C_6$, un $(CH_2)_n$-phényle où n peut valoir de 0 à 6 et le radical phényle peut être jusqu'à bi-substitué par F, Cl, Br, $CF_3$, CN, $OCF_3$, un O($C_1$-$C_6$)alkyle, un S($C_1$-$C_6$)-alkyle, un alkyle en $C_1$-$C_6$, un cycloalkyle en $C_3$-$C_6$, un COO($C_1$-$C_6$)alkyle, un COO($C_3$-$C_6$)cycloalkyle, un $CONH_2$, un CONH($C_1$-$C_6$)alkyle, un CON [($C_1$-$C_6$)-alkyle]$_2$ ;

R3 représente NR10R11, une pipérazine ;

R10, R11 représentent indépendamment l'un de l'autre, H, un alkyle en $C_1$-$C_6$, un cycloalkyle en $C_3$-$C_6$, un (CO)-($C_1$-$C_6$) alkyle, un ($C_1$-$C_6$)alkyl-CO-($C_1$-$C_6$)-alkyle, un $SO_2$-benzyle, un $SO_2$-benzyl-$OCH_3$, un $(CH_2)_n$-Ar, où n peut valoir de 0 à 6 et Ar peut être un phényle ou un thiényle ;

ainsi que leurs sels physiologiquement acceptables.

**2.** Médicament contenant un ou plusieurs des composés selon la revendication 1.

**3.** Médicament contenant un ou plusieurs des composés selon la revendication 1 et un ou plusieurs hypolipémiants.

**4.** Composés selon la revendication 1 destinés à être utilisés en tant que médicament pour le traitement de l'hyperlipidémie.

**5.** Composés selon la revendication 1 destinés à être utilisés en tant que médicament pour le traitement de l'artériosclérose.

**6.** Procédé de production d'un médicament contenant un ou plusieurs des composés selon la revendication 1, **caractérisé en ce que** l'ingrédient actif est mélangé avec un support pharmaceutiquement approprié et ce mélange est mis sous une forme appropriée pour l'administration.

**7.** Utilisation des composés selon la revendication 1 pour la production d'un médicament destiné au traitement de l'hyperlipidémie.

**8.** Utilisation des composés selon la revendication 1 pour la production d'un médicament destiné au traitement de l'artériosclérose.